# EUROPEAN PATENT APPLICATION

(11) **EP 1 206 947 A1**
(43) Date of publication of application: **22.05.2002**
(21) Application number: 01305572.8
(22) Date of filing: 27.06.2001
(51) Int. Cl.: A61M 5/32, A61M 25/06, B22D 17/00

(54) **One-piece needle**

(30) Priority: 29.06.2000 US 608671
(71) Applicant: ETHICON, INC., Somerville, NJ 08876 (US)
(72) Inventor: Kafrawy, Adel, Kingston, MA 02364 (US); Schmidt, Phillip, Arlington TX 76018 (US)
(74) Representative: Mercer, Christopher Paul

(57) **Abstract**

A method and an apparatus is disclosed for forming a one-piece introducer needle having a member portion and a needle by feeding molten steel into a cavity of a mold.

## Description

### BACKGROUND OF THE INVENTION

### Field of the Invention

This invention relates generally to intravascular assemblies, and more specifically to a one-piece introducer needle and method of making the needle.

### Background

Intravascular devices such as catheter assemblies are generally used for passing fluids between a device such as a syringe or a drip to or from body lumens such as veins or arteries, or other internal target sites. Such an assembly usually includes a hub, and a catheter tube. The tube is typically secured to the hub by means of an eyelet ring that is press fit within the nose of the hub. This hub and tube assembly is then mounted over an introducer needle comprising a sharp needle attached to a plastic hub. The sharp tip of the needle, protruding from the catheter tip, is used for piercing a body lumen so that access may be gained into the body lumen by the needle and subsequently the catheter. Once the catheter and the needle are located within the body lumen, the introducer needle is removed and discarded while the catheter tube remains in the body lumen. A syringe or a tube of a drip is then attached to the hub so that fluids may be passed through the hub and the catheter from the drip or the syringe to the body lumen. The hub is typically made of materials that provide sufficient rigidity thereto and the catheter tube is usually made of a material which is flexible.

Intravenous introducer needles with a surface groove are known in the art. One purpose of intravenous introducer needles is to allow a healthcare worker to be able to quickly observe when back-flow of blood enters a surface groove indicating that the needle has penetrated the vein. Although introducer needles are known, there are several disadvantages to introducer needles such as those described in U.S. Patent No. 5,279,572, issued to Hokam (*"Hokam"*), and European Patent No. EPO 893 137 A2, issued to Terumo Kabushiki Kaisha. For example, *Hokam* comprises an intravenous introducer needle having two blood back-flow passage routes wherein the needle comprises a material that is made of steel. The needle and the handle are manufactured separately through a machining operation and thereafter are fastened together. By requiring that a stainless steel needle be joined to a needle base, an assembling cost is incurred. It is therefore desirable to have a method of fabricating a one-piece needle that is able to reduce the costs associated with conventional devices such as the operational cost such as machining of the needle and handle, and the cost of assembling these elements.

### SUMMARY

An apparatus and a method are disclosed for forming a one-piece introducer needle having a head. The method comprises feeding molten steel into a cavity of a mold. The first portion of the cavity forms a head and the second portion of the cavity forms a needle. Additional features, embodiments, and benefits will be evident in view of the figures and detailed description presented herein.

### BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention.

**Figure 1** illustrates a mold comprising three sections which are mated together and molten steel is shown entering the cavity in accordance with one embodiment of the invention.

**Figure 2** illustrates the same mold as **Figure 1,** wherein the molten steel has moved further in the distal direction of the cavity of the mold in accordance with one embodiment of the invention.

**Figure 3** illustrates the same mold as **Figure 2** wherein the molten steel has almost filled the cavity in accordance with one embodiment of the invention.

**Figure 4** illustrates the same mold as in **Figure 3** wherein the molten steel has filled the cavity in accordance with one embodiment of the invention.

**Figure 5** illustrates a one-piece needle inserted into a catheter and hub in accordance with one embodiment of the invention.

**Figure 6** illustrates an isometric view of blood entering a surface groove of a solid tip-surface grooved needle in accordance with one embodiment of the invention.

**Figure 7** illustrates an isometric view of blood entering a surface groove of a solid tip-surface grooved needle inserted into a catheter tube in accordance with one embodiment of the invention.

**Figure 8** illustrates a top view of blood entering a surface groove of a solid tip-surface grooved needle in accordance with one embodiment of the invention.

**Figure 9** illustrates an isometric view of blood entering a surface groove of a hollow bored tip needle in accordance with one embodiment of the invention.

**Figure 10** illustrates a top view of blood entering a surface groove of a hollow bored tip needle in accordance with one embodiment of the invention.

**Figure 11** illustrates an isometric view of blood entering a hollow needle bore tip and a surface groove in accordance with one embodiment of the invention.

**Figure 12** illustrates a top view of blood entering a hollow bore tip needle and a surface groove in accordance with one embodiment of the invention.

**Figure 13** illustrates a side view of a solid tip needle inserted into a patient's vein and blood entering the distal end of the surface groove in accordance with one embodiment of the invention.

**Figure 14** illustrates a top view of a solid tip needle inserted into a patient's vein and blood entering the distal end of the surface groove in accordance with one embodiment of the invention.

**Figure 15** illustrates a side view of a needle having a hollow bore tip and a surface groove wherein blood enters the hollow bore of the needle in accordance with one embodiment of the invention.

**Figure 16** illustrates a top view of a needle with a hollow bore tip wherein blood enters the hollow bore of the needle in accordance with one embodiment of the invention.

**Figure 17** illustrates a mold in accordance with one embodiment of the invention.

**Figure 18** illustrates a mold of **Figure 17** in which the molten steel has moved further in the direction of various portions of the mold in accordance with one embodiment of the invention.

**Figure 19** illustrates the molten steel having filled portions of the mold in accordance with one embodiment of the invention.

**Figure 20** illustrates the molten steel having filled the distal portion of one-piece needle cavity in accordance with one embodiment of the invention.

**Figure 21** illustrates the one-piece needle formed from the process illustrated in **Figures 17-20** inserted into a catheter and hub cavity in accordance with one embodiment of the invention.

**Figure 22** illustrates a mold to form a one-piece spiral needle in accordance with one embodiment of the invention.

**Figure 23** illustrates molten steel continuing to fill the mold in accordance with one embodiment of the invention.

**Figure 24** illustrates the molten steel having filled the mold in accordance with one embodiment of the invention.

**Figure 25** illustrates the one-piece spiral needle inserted into a catheter and hub in accordance with one embodiment of the invention.

**Figure 26** illustrates a mold in accordance with one embodiment of the invention.

**Figure 27** illustrates the molten steel using the same mold as **Figure 26** entering portion and continuing down the distal portion in accordance with one embodiment of the invention.

**Figure 28** illustrates that the molten steel has filled the mold in accordance with one embodiment of the invention.

**Figure 29** illustrates the one-piece needle formed from the process illustrated in **Figures 26-29** locked in place by using a catheter and hub that has a recessed region for receiving a portion of the one-piece needle in accordance with one embodiment of the invention.

**Figure 30** illustrates a mold in accordance with one embodiment of the invention.

**Figure 31** shows the molten steel has filled the head portion of the one-piece needle.

**Figure 32** illustrates the same mold as **Figure 31** with the molten steel having filled the cavity of the one-piece needle.

**Figure 33** illustrates the one-piece needle formed in process shown in **Figures 30-32** inserted into a hub and catheter.

**Figure 34** illustrates a mold comprising three sections that are mated together and molten steel is shown entering the cavity in accordance with one embodiment of the invention.

**Figure 35** illustrates the same mold as **Figure 34** wherein the molten steel has moved further in the distal direction of the cavity of the mold in accordance with one embodiment of the invention.

**Figure 36** illustrates the same mold as **Figure 35** wherein the molten steel has filled the cavity of the mold in accordance with one embodiment of the invention.

**Figure 37** illustrates a one-piece needle inserted into a catheter and hub in accordance with one embodiment of the invention.

**Figure 38** illustrates a cross-section of a solid tip needle with a surface groove in accordance with one embodiment of the invention.

**Figure 39** illustrates a cross-section of a hollow bored tip needle with a surface groove in accordance with one embodiment of the invention.

**Figure 40** illustrates a cross-section of a solid tip needle with a rectangular shaped surface groove in accordance with one embodiment of the invention.

**Figure 41** illustrates a cross-section of a hollow tip needle with a rectangular shaped surface groove in accordance with one embodiment of the invention.

**Figure 42** illustrates a cross-section of a solid tip needle with a v-shape surface groove in accordance with one embodiment of the invention.

**Figure 43** illustrates a cross-section of a hollow tip needle with a v-shape surface groove in accordance with one embodiment of the invention.

**Figure 44** illustrates a cross-section of a solid tip needle with a surface groove having a half circle shape in accordance with one embodiment of the invention.

**Figure 45** illustrates a cross-section of a hollow tip bored needle having a half circle shape surface groove in accordance with one embodiment of the invention.

**Figure 46** illustrates a cross-section of a solid tip needle with a plurality of surface grooves having a half circle shape in accordance with one embodiment of the invention.

**Figure 47** illustrates a cross-section of a solid tip needle with a plurality of surface grooves having a substantially v-shape in accordance with one embodiment of the invention.

**Figure 48** illustrates a cross-section of a hollow bore tip needle with a surface groove inserted into a catheter tube in accordance with one embodiment of the invention.

### DETAILED DESCRIPTION

One embodiment of the invention relates to forming a one-piece steel introducer needle for use in an intravascular assembly. Another embodiment of the invention relates to the formation of a beveled sharp end of the distal tip of the one-piece needle.

Referring to the figures, exemplary embodiments of the invention will now be described. The exemplary embodiments are provided to illustrate aspects of the invention and should not be construed as limiting the scope of the invention.

Presented below is a description of the materials that may be used followed by a process description of forming a one-piece needle. It will be appreciated that the one-piece needle may have a hollow bored or solid tip. Additionally, a surface groove may be formed in the one-piece needle. Next, several embodiments are presented of the various molds that may be used to form a variety of one-piece needles. Thereafter, cross-sections of the one-piece needle is presented that show a variety of different shaped surface grooves that may be formed in the one-piece needle.

Steel may be used for molding of the one-piece introducer needle. Steel may have a variety of characteristics including those characteristics shown in Table I and Table II. However, other grades of steel may also be used to form one-piece needles.

**Table I.**

| Chemical and nominal mechanical properties of austenitic and ferritic stainless steels. | | | | | | | | | | | | | |
|---|---|---|---|---|---|---|---|---|---|---|---|---|---|
| Chemical Analysis % (maximum unless noted) | | | | | | | | | | TS (ski) | Ys (ksi) | EL (%) | Hardness (Rockwell) |
| AISI | C | Mn | P | S | Si | Cr | Ni | Mo | Other | | | | |
| Austenitic Chromium-Nickel Steel | | | | | | | | | | Annealed Condition | | | |
| 301 | 0.15 | 2 | 0.045 | 0.03 | 1.00 | 16.00-18.00 | 6.00-8.00 | | | 110 | 40 | 60 | Rb 85 |
| 303 | 0.15 | 2 | 0.2 | 0.15 min. | 1.00 | 17.00-19.00 | 8.00-10.00 | 0.6 | | 90 | 35 | 50 | - |
| 304 | 0.08 | 2 | 0.045 | 0.03 | 1.00 | 18.00-20.00 | 8.00-10.50 | | | 84 | 42 | 55 | Rb 80 |
| 304L | 0.03 | 2 | 0.045 | 0.03 | 1.00 | 18.00-20.00 | 8.00-12.00 | | | 81 | 39 | 55 | Rb 79 |
| 310 | 0.25 | 2 | 0.045 | 0.03 | 1.5 | 24.00-26.00 | 19.00-22.00 | | | 95 | 45 | 45 | Rb 85 |
| 310S | 0.08 | 2 | 0.045 | 0.03 | 1.5 | 24.00-26.00 | 19.00-22.00 | | | 95 | 45 | 45 | Rb 85 |
| 316 | 0.08 | 2 | 0.045 | 0.03 | 1.00 | 16.00-18.00 | 10.00-14.00 | 2.00-3.00 | | 84 | 42 | 50 | Rb 79 |
| 316L | 0.03 | 2 | 0.05 | 0.03 | 1.00 | 16.00-18.00 | 10.00-14.00 | 2.00-3.00 | | 81 | 42 | 50 | Rb 79 |
| 317 | 0.08 | 2 | 0.045 | 0.03 | 1.00 | 18.00-20.00 | 11.00-15.00 | 3.00-4.00 | | 90 | 40 | 45 | Rb 85 |

| Ferritic Nonhardenable Stainless | | | | | | | | | | Annealed Condition | | | |
|---|---|---|---|---|---|---|---|---|---|---|---|---|---|
| 430 | 0.12 | 1.00 | 0.04 | 0.03 | 1.00 | 16.00-18.00 | | | | 75 | 50 | 25 | Rb 85 |
| 446 | 0.2 | 1.5 | 0.04 | 0.03 | 1.00 | 23.00-27.00 | | | 0.25 N | 80 | 50 | 20 | Rb 83 |

**Table II.**

| Chemical and nominal mechanical properties of martensitic and precipitation-hardening stainless steels. | | | | | | | | | | | | | |
|---|---|---|---|---|---|---|---|---|---|---|---|---|---|
| Chemical Analysis % (maximum unless noted) | | | | | | | | | | TS (ksi) | Ys (ksi) | EL (%) | Hardness (Rockwell) |
| AISI | C | Mn | P S | | Si | Cr | Ni | Mo | Other | | | | |
| Martensitic Chromium Steel | | | | | | | | | | Annealed (Hardened) | | | |
| 410 | 0.15 | 1.00 | 0.04 | 0.03 | 1.00 | 11.50-13.00 | | | | 70 | 45 | 25 | Rb 80 |
| 420 | 0.15 min. | 1.00 | 0.04 | 0.03 | 1.00 | 12.00-14.00 | | | | 95 | 50 | 25 | Rb 92 |
| 431 | 0.2 | 1.00 | 0.04 | 0.03 | 1.00 | 15.00-17.00 | 1.25-2.50 | | | 125 | 95 | 20 | Rc 24 |
| 440A | 0.60/0.75 | 1.00 | 0.04 | 0.03 | 1.00 | 16.00-18.00 | | 0.75 | | 105 | 60 | 20 | Rb 95 |
| 440B | 0.75/0.95 | 1.00 | 0.04 | 0.03 | 1.00 | 16.00-18.00 | | 0.75 | | 107 | 62 | 18 | Rb 96 |
| 440C | 0.95/1.20 | 1.00 | 0.04 | 0.03 | 1.00 | 16.00-18.00 | | 0.75 | | 110 | 65 | 14 | Rb 97 |
| Trim Rite™ | 0.15/1.30 | 1.00 | 0.04 | 0.03 | 1.00 | 13.50-15.00 | 0.25-1.00 | 0.40-1.00 | | 88 | 54 | 28 | Rb 88 |

| Precipitation-Hardening Stainless Steels | | | | | | | | | | Solution-Annealed | | | |
|---|---|---|---|---|---|---|---|---|---|---|---|---|---|
| 13-8 | 0.05 | 0.1 | 0.01 | 0.008 | 0.1 | 12.25-13.25 | 7.50-8.50 | 2.00-2.50 | 0.9/1.35Al 0.010 N | 160 | 120 | 17 | Rc 33 |
| 15-5 | 0.07 | 1.00 | 0.04 | 0.03 | 1.00 | 14.00-15.50 | 3.50-5.50 | | 2.5/4.5 Cu 0.15/0.45 Cb Ta | 160 | 145 | 15 | Rc 35 |
| 17-4 | 0.07 | 1.00 | 0.04 | 0.03 | 1.00 | 15.50-17.50 | 3.00-5.00 | | 3.0/5.0 Cu 0.15/0.45 Cb Ta | 160 | 145 | 15 | Rc 35 |
| 17-7 | 0.09 | 1.00 | 0.04 | 0.04 | 0.04 | 16.00-18.00 | 6.50-7.75 | | 0.75/1.50 Al | 130 | 40 | 10 | Rb 90 |
| 455 | 0.05 | 0.5 | 0.04 | 0.03 | 0.5 | 11.00-12.50 | 7.50-9.50 | 0.5 | 15/2.5 Cu 0.8/1.4 Ti 0.1/0.5 Cb Ta | 145 | 115 | 14 | Rc 31 |
| ™This is a product sold by Carpenter Technology Corporation located in Reading, Pennsylvania | | | | | | | | | | | | | |

There are a variety of methods that may be used in forming a one-piece steel needle such as continuous casting. In a continuous casting method, steel from an electric furnace, basic oxygen furnace or other like furnace is tapped into a container and taken to a continuous casting machine. The container is raised onto a turret that rotates the container into the casting position above the tundish. Liquid steel flows out of the container into tundish and then into a water-cooled copper mold. Solidification begins in the mold.

The mold bottom may be sealed by a steel bar that is held in place hydraulically by a device such as a straightener withdrawal unit. This steel bar prevents liquid steel from flowing out of the mold. The steel placed into the mold begins to solidify immediately. Liquid steel continues to be placed into the mold until the mold cavity is filled with steel.

The mold provides a solid shell sufficient in strength to contain its liquid core upon entry into the secondary spray cooling zone. The mold may be an open-ended structure containing a water-cooled inner lining fabricated from a high purity copper alloy. Water is used to transfer heat from the solidifying shell. The working surface of the copper face of a mold may be plated with chromium, nickel or like material to provide a harder working surface. It will be appreciated that other conventional processes may be used to form a one-piece steel needle such as injection molding. Given the description of some of the types of steel that may be used and conventional processes used to manufacture the one-piece needle, descriptions of the types of molds used are presented below.

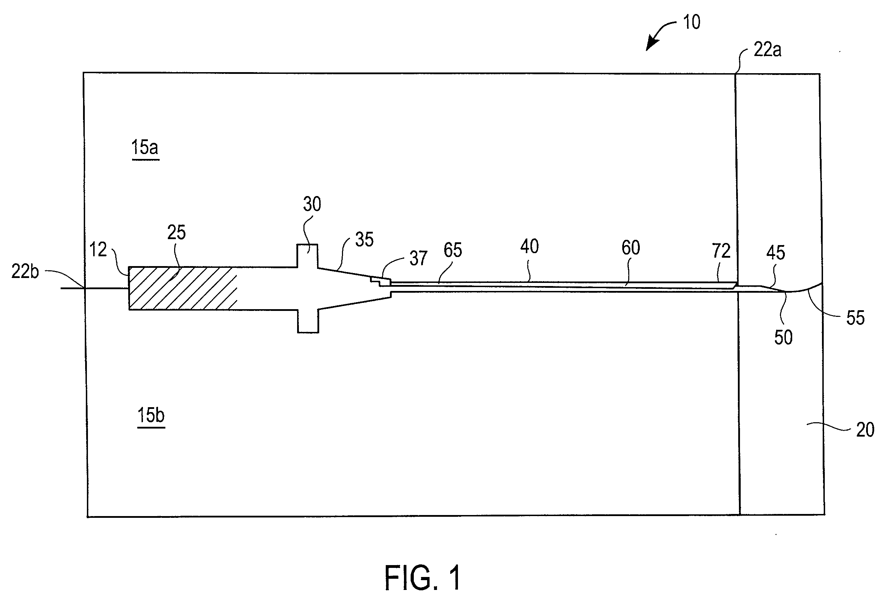

**Figures 1-5** illustrate one embodiment of the invention molding a one-piece needle. **Figure 1** illustrates mold 10 comprising first and second sections 15a and 15b and third section 20 that are mated together. The cavity formed by mold 10 comprises a head in the shape of a handle 25, a flange 30 portion of the handle, a tapered portion 35 of handle 25, a stepped portion 37 of the handle 25, a needle 40, wherein the needle portion has a surface groove 60, and a beveled portion 55 that transitions to a distal sharp tip 50 of needle 40. Surface groove 60 extends from proximal end 65 of surface groove 60 to the distal end 72 of the groove channel.

Mold 10 has an inlet 12 that allows molten steel to enter mold 10. The molten steel is introduced at inlet 12 of mold 10 at a pressure in the approximate range of 1 psi to 100 psi. Additionally, the molten steel is generally maintained at a temperature that ranges from 1600°F to 2200°F. It will be appreciated that other pressures and temperatures may be used depending upon the grade of steel selected. The molten steel begins to move through handle 25 in the direction of the distal end of handle 25.

The dimensions of the cavity that is formed from sections 15a, 15b, and 20 varies with the gauge of the intravascular assembly to be fabricated. In this embodiment, a hollow bored tip needle with a surface groove is formed. The outer diameter of the handle may range from approximately 0.25 inches to 0.35 inches in the proximal portion of the handle. The inner diameter of the proximal portion of handle 25 may range from approximately 0.20 inches to 0.30 inches. The outer diameter of flange 30 may range from approximately 0.50 inches to 0.80 inches. The inner diameter of flange 30 may range from approximately 0.40 inches to 0.60 inches. Tapered portion of handle 25 has an inner and an outer diameter that varies with the tapered portion of a particular needle being formed. However, the range of outer diameters of the tapered portion may range from 0.20 inches to 0.30 inches. Similarly, the inner diameter of the tapered portion may range approximately from 0.15 inches to 0.25 inches. The first stepped portion of handle 25 has an outer diameter that ranges from approximately 0.30 inches to 0.40 inches and the inner diameter ranges from approximately 0.25 inches to 0.35 inches. Second stepped portion of handle 25 has an outer diameter that ranges from 0.25 inches to 0.35 inches and an inner diameter that ranges from approximately 0.20 inches to 0.30 inches, and a length that ranges from approximately 0.25 inches to 0.75 inches. Needle 40 may have a length that ranges from approximately 0.70 inches to 2.5 inches, an outer diameter that ranges from approximately 0.10 inches to 0.02 inches. It will be appreciated that a solid tip needle lacks the inner diameter dimensions listed for the proximal portion of handle 25, flange 30, the tapered portion of handle 25, the first and second stepped portions of handle 25.

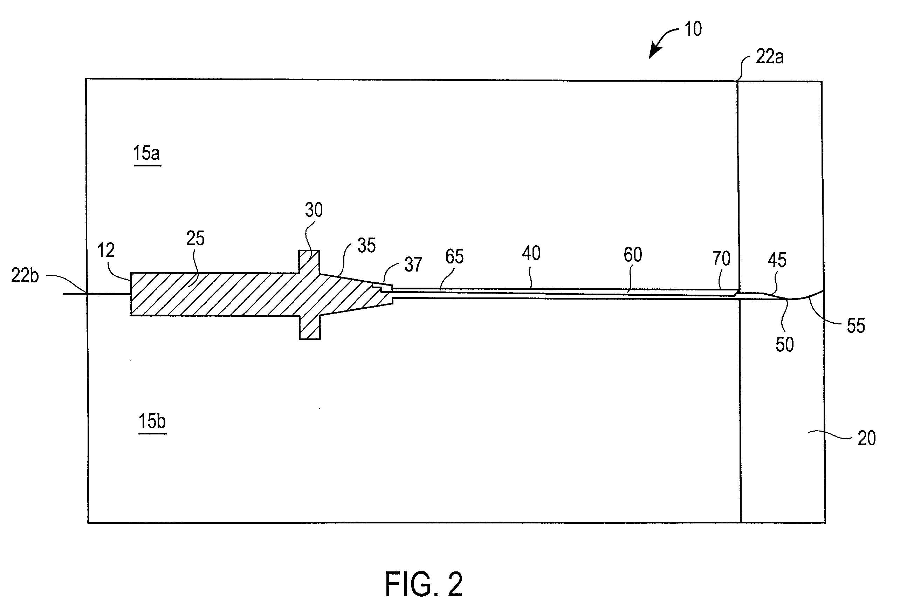

In this embodiment, needle 40 that is formed has a solid tip but a surface groove is formed therein. (It will be appreciated that hollow bored tip needles may be formed also using a different configuration of a mold such as by using a core pin at the distal end of needle 40.) Surface groove 60 allows blood to enter needle 40 at or near the distal end of needle 40. The blood moves in the proximal direction of needle 40 as shown in **Figure 1.** It will be appreciated that the surface groove channel in **Figure 1** illustrates that since the surface groove channel is formed on the external portion of the needle, there is no outer diameter for the surface groove channel. The distance from proximal end 65 and distal end 72 of surface groove 60 ranges from approximately 0.25 inches to 2.5 inches. **Figure 2** illustrates the same mold as **Figure 1,** however, the molten steel has moved further in the distal direction of the cavity.

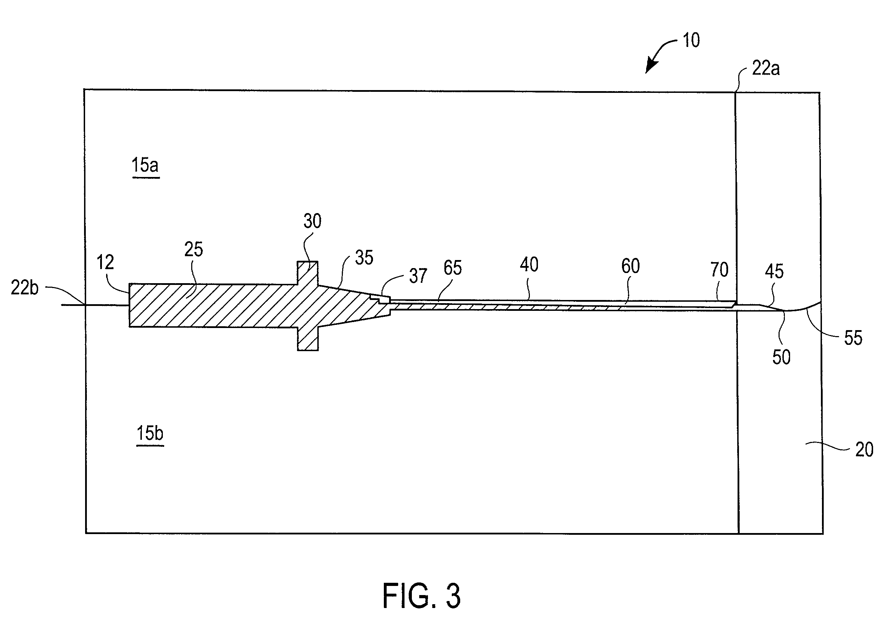

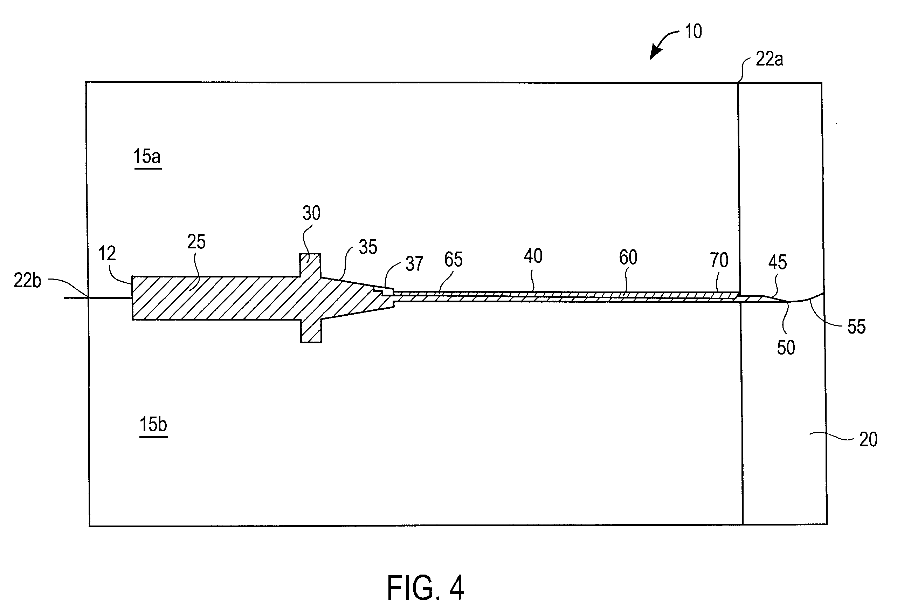

**Figure 2** illustrates molten steel continues to be introduced at inlet 12 of mold 10. The molten steel moves in the direction from the base of the one-piece introducer needle toward the distal portion of needle 40. **Figure 3** illustrates mold 10 of **Figure 2** in which the molten steel has filled handle 25 and is beginning to move into the cavity for needle 40. **Figure 4** illustrates mold 10 of **Figure 3** wherein the molten steel is advanced such that the cavity is filled, thereby forming a one-piece introducer needle. The mold opens at parting lines 22a and 22b. Once the mold is opened, the one-piece introducer needle is then removed from mold 10 using conventional techniques. In another embodiment, the manufacturer of the one-piece needle may choose after the secure, attach, couple, or fasten an additional metallic tip to the distal tip of needle 40 using conventional techniques.

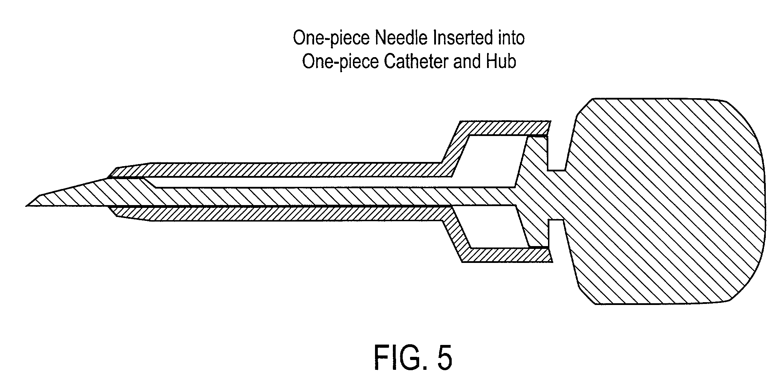

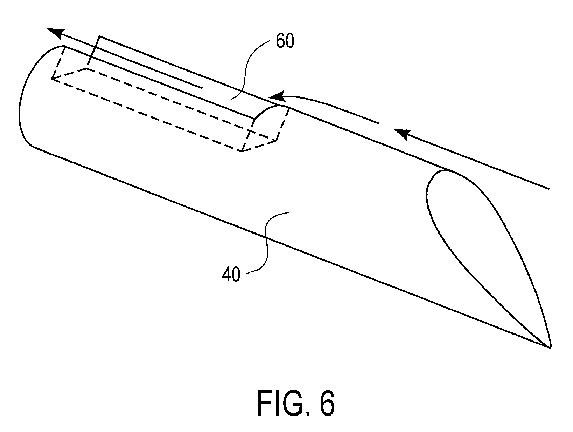

**Figure 5** illustrates the one-piece needle formed from the process shown in **Figures 1** through **4** inserted into a catheter and hub. It will be appreciated that the one-piece needle tip formed from this process may be either hollow or solid. Additionally, the one-piece needle may have a surface groove. The surface groove may have a variety of shapes as illustrated in **Figures 6** to **16, 22** to **25,** and **38** to **48.**

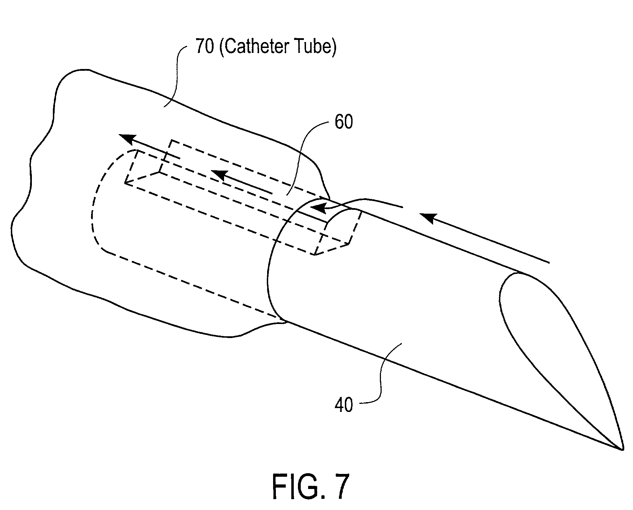

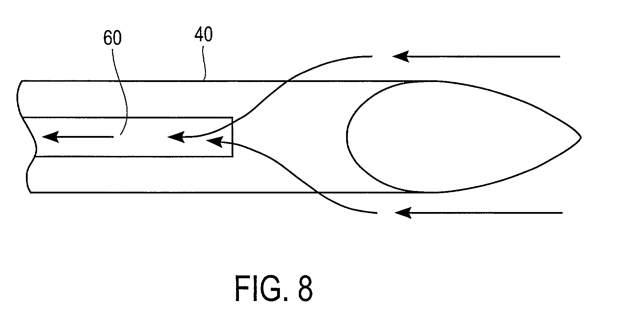

**Figures 6** through **8** illustrate one embodiment of the invention in which a needle 40 has a solid tip portion and a surface groove 60. **Figures 6** and **7** provide isometric views of solid needle 40 and shows the flow of blood passing over the distal tip of needle 40 and entering surface groove 60. **Figure 7** further illustrates the flow of blood when needle 40 is coupled to a catheter tube 70. **Figure 8** illustrates a top view of needle 40 wherein blood flows around solid tip needle 40 and enters surface groove 60.

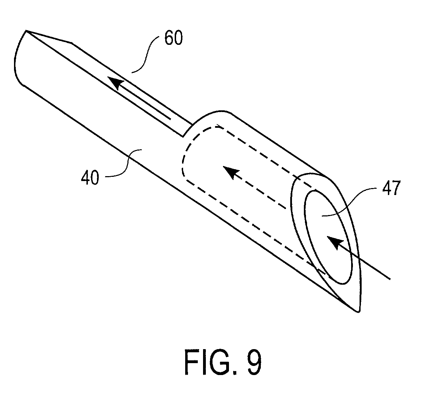

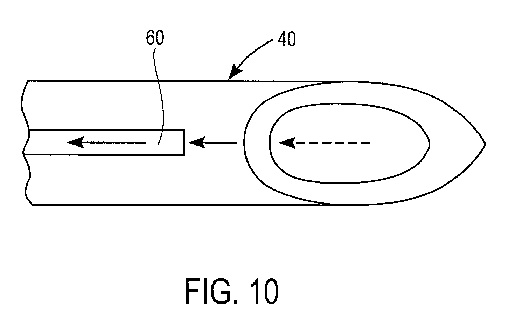

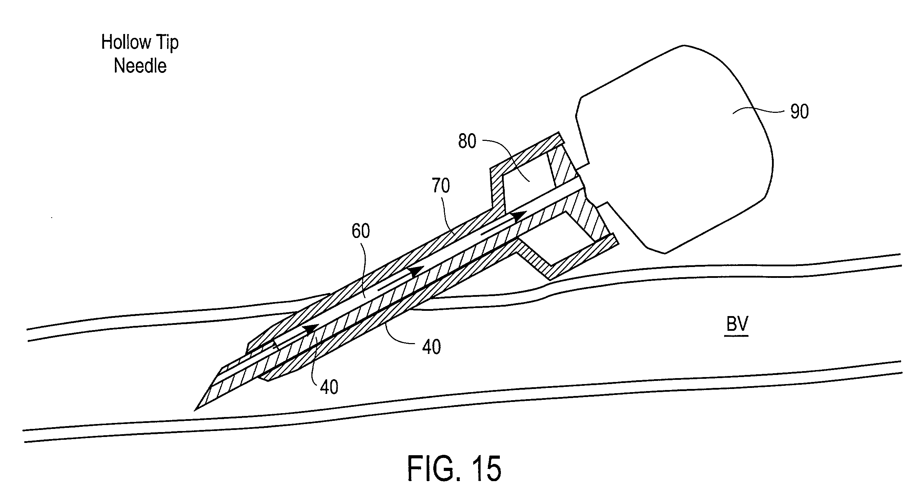

**Figure 9** provides an isometric view and **Figure 10** illustrates a top view of the flow of blood through hollow bored needle 40. Surface groove 60 does not extend the length of needle 40 as illustrated in **Figures 9** and **10**. The hollow bore section 47 is associated with the distal tip and is approximately in the range of 0.15 and 0.5 inches, and preferably 0.25 inches long. The distal end of the hollow bore section 47 contains a molded distal tip while the proximal end of the hollow bore section transitions to a solid rod located off-center toward the bottom of the device. Hollow bore section 47 provides a passage for the blood from the tip to an opening at the proximal end of the hollow bore of needle 40. The hollow bore of needle 40 is formed by designing a core pin in the mold cavity which will core out the distal end of the needle. During insertion of the distal tip of needle 40 into a patient, blood passes through the distal tip into the hollow bore section 47 of the needle 40 and fills the volume defined by surface groove 60 of the needle and the catheter tube 70 as seen in **Figure 15.** An optically clear catheter tube allows a user to observe flashback of blood or other fluids into the catheter tube.

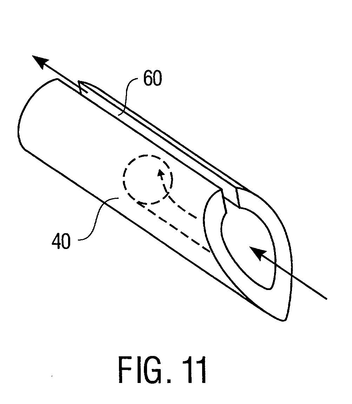

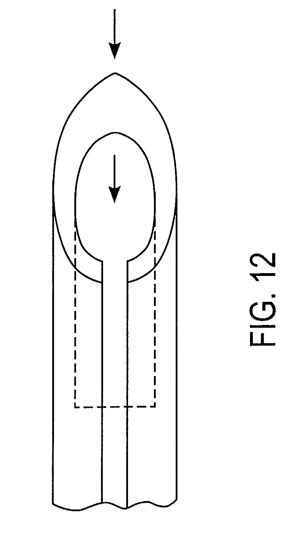

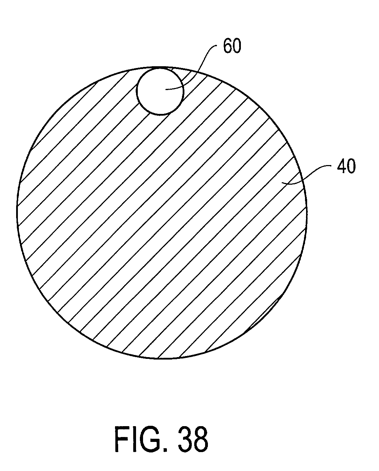

**Figures 11** and **12** illustrate another embodiment of the invention in which needle 40 has a hollow bore tip and a surface groove 60 that extends the length of needle 40. **Figure 11** provides an isometric view of needle 40 with a hollow bore tip therein and surface groove 60 and **Figure 12** illustrates a top view of needle 40 with a hollow bore tip and surface groove 60. Blood may enter both the hollow bore tip of the needle and the surface groove at about the same time or the blood may enter the hollow bore tip and then enter surface groove 60 of needle 40. Surface groove 60 may be of a variety of geometries such as that which is shown in **Figures 38** through **48.**

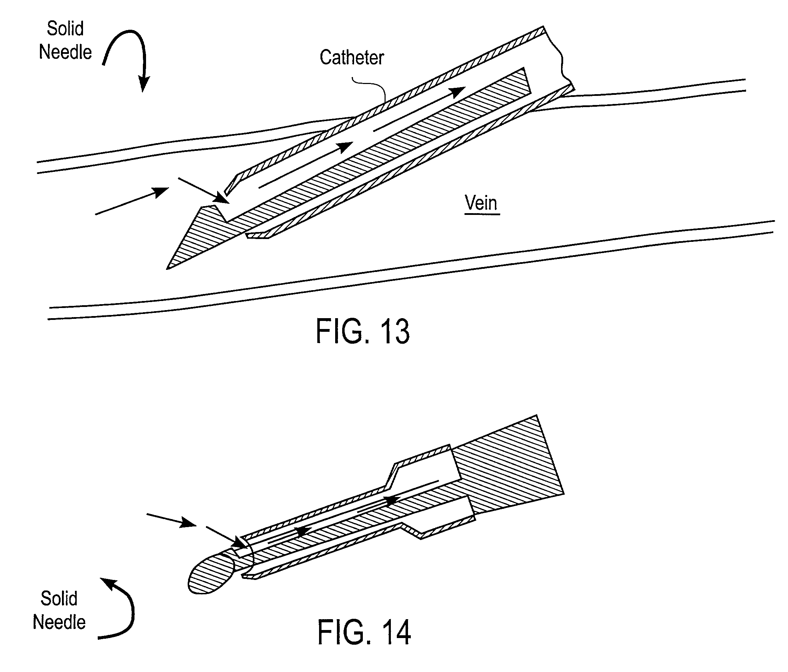

**Figures 13** and **14** illustrate needle 40 and needle tip with a pointed end inserted into a patient's vein. The needle tip is a solid sharp. The blood flows into the surface groove and travels to the proximal end of needle 40.

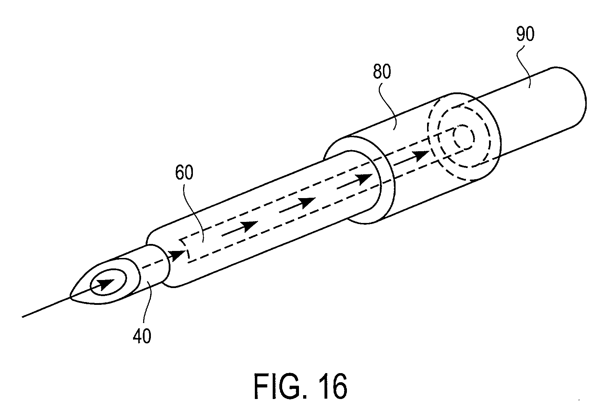

**Figures 15** and **16** illustrate needle 40 and head 90 inserted into catheter tube 70 with catheter hub 80. Needle 40 has a hollow bore and a surface groove. Blood may pass through the bore of needle 40 and then enter the surface groove. Alternatively, with the surface groove extended to the tip, as in **Figure 11,** the blood may enter the surface groove at about the same time it enters the bore of needle 40. The blood may then travel toward needle head (or handle) 90, flowing between surface groove 60 and catheter tube 70.

Given the explanation of a surface groove in the one-piece needle, the following description presents a variety of molds that may be used to form other one-piece needles with a variety of different shaped heads and one-piece needles with and without surface grooves.

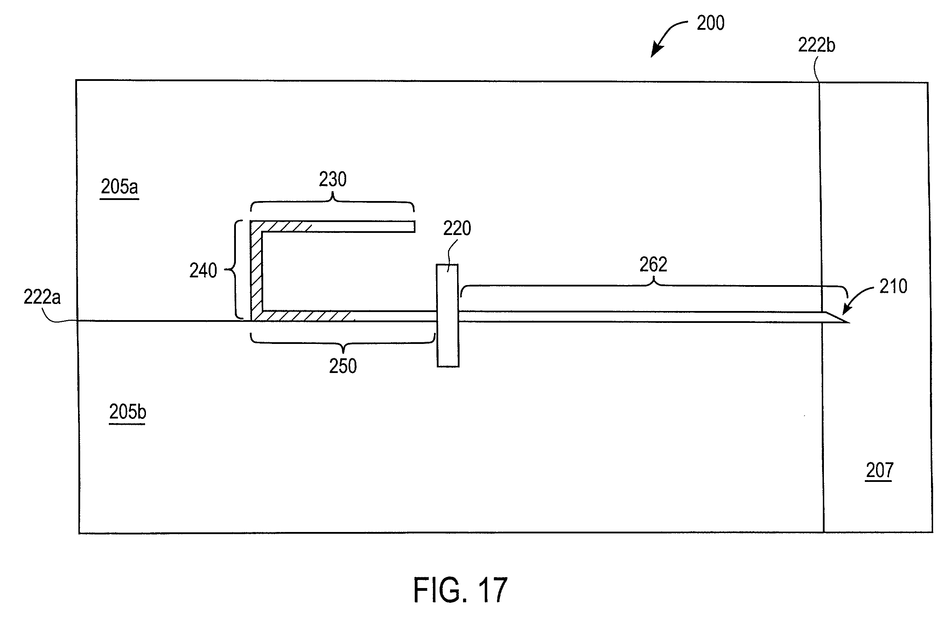

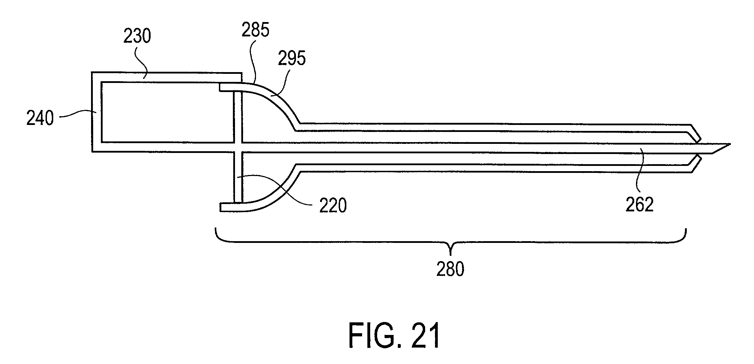

**Figures 17** through **21** illustrate one embodiment of the invention using injection molding to form a one-piece needle. **Figure 17** illustrates mold 200 in accordance with one embodiment of the invention. Mold 200 comprises first and second sections 205A and 205B and third section 207 mated together. The cavity formed by mold 200 comprises a substantially rectangular portion at the proximal end formed by portions 230, 240, and 250. Disk portion 220 is located at the proximal end of the one-piece needle. One purpose of disk portion 220 is locking the one-piece needle into a catheter and hub as illustrated in **Figure 21.** The one-piece needle then transitions into a distal portion of one-piece needle 262. Mold 200 further comprises a beveled distal tip 210 at the distal end of the one-piece needle. Mold 200 has an inlet 222a that allows molten steel to enter mold 200. The molten steel is introduced at inlet 208 of mold 200 at a pressure in the approximate range of 1 psi to 100 psi. Additionally, the molten steel generally is maintained at a temperature that ranges from 1600°F to 2200°F. It will be appreciated that other pressures and temperatures may be used depending upon the material selected. The steel begins to move through portion 240 in the direction of portions 230 and 250.

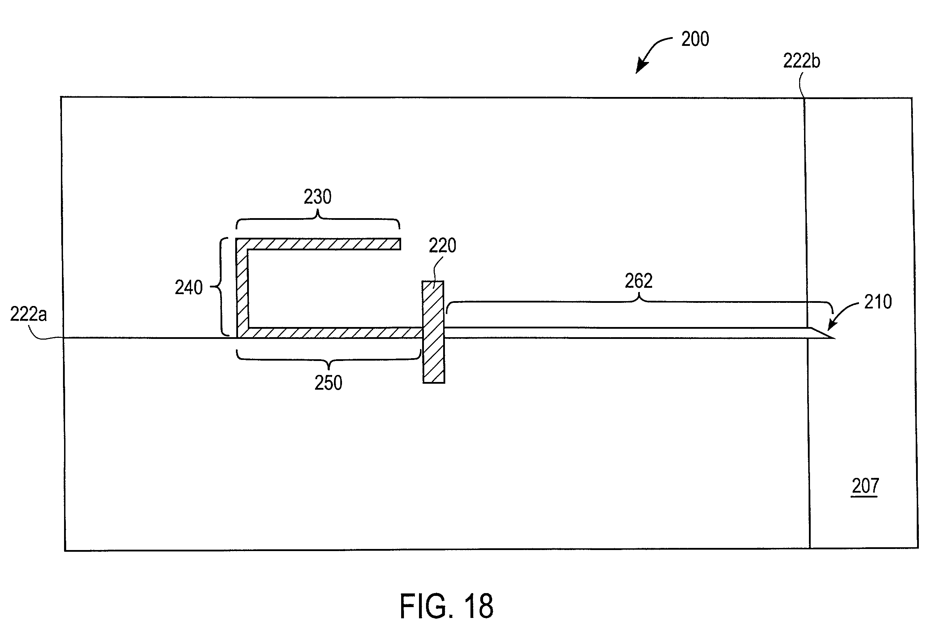

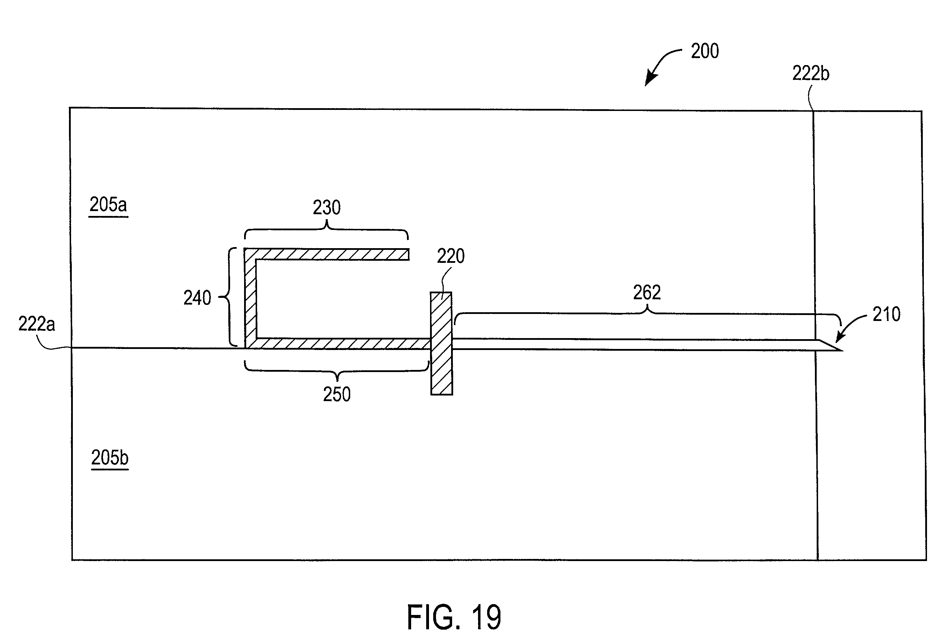

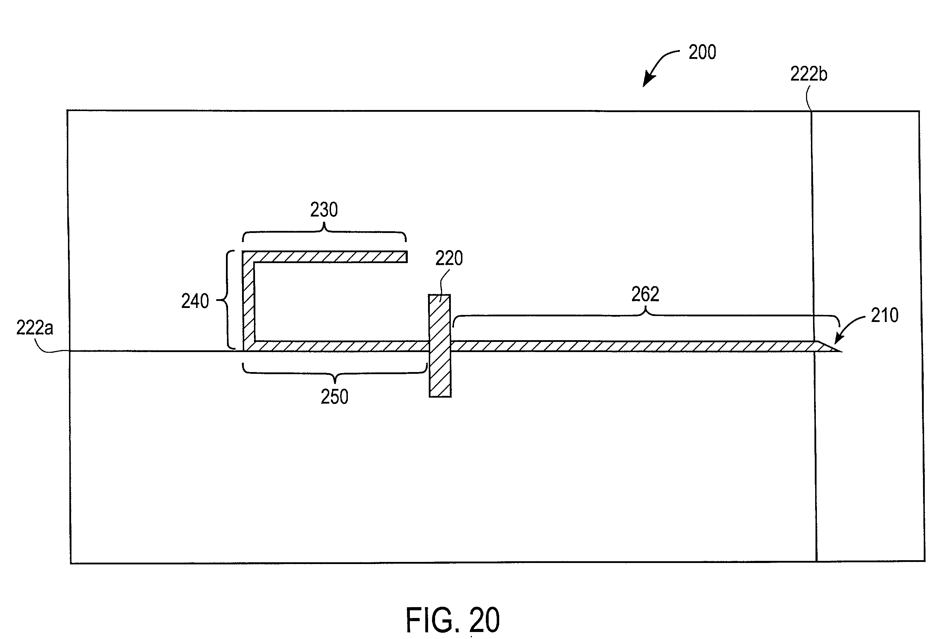

It will be appreciated that the dimension of the one-piece introducer needle that is formed from sections 205A, 205B and 207 vary with the gauge of the intravascular assembly to be fabricated. For example, the distal portion of one-piece needle 262 may range in length from 0.7 inches to 2.5 inches. Furthermore, disk portion 220 may range in diameter from 0.15 inches to 0.35 inches. This large range is present to accommodate the design of a luer lock feature in the hub. Portion 250 may range from 0.5 inches to 2.0 inches. Portion 240 may range from 0.5 inches to 2.0 inches. Portion 230 may range from 0.5 inches to 2.0 inches. It will be appreciated that the diameter of the needle is the same as that which is described above and is dependent upon the gauge of the intravascular assembly to be fabricated. **Figure 18** illustrates the mold as **Figure 17,** wherein the steel has moved further in the direction of portions 230 and 250. **Figure 19** illustrates the mold of **Figure 18** with the molten steel having filled cavity portions 230, 240, and 250 and disk portion 220. **Figure 20** illustrates the molten steel having filled the distal portion of one-piece needle 262. The mold opens at parting lines 222a and 222b. Once the mold is opened, the one-piece introducer needle is then removed from mold 200 using conventional techniques. In another embodiment, after the one-piece introducer needle has been formed, a metallic needle tip may be secured, attached, coupled, or fastened to the distal tip of the needle using conventional techniques.

**Figure 21** illustrates the one-piece needle formed from the process illustrated in **Figures 17-20** inserted into a catheter and hub 280. It will be appreciated that the one-piece needle formed from the process illustrated in **Figures 17-20** is snapped into place at block 285 that has a recessed region for receiving portion 230. It will also be appreciated that disk portion 220 is locked in place at block 285 that also has a recessed region within the inner surface of hub 295. When the one-piece needle snaps into place, a noise is emitted such as a clicking noise. This clicking noise indicates to the healthcare worker that the needle is locked in place. The blood flows from the distal portion of one-piece needle 262 and flows toward disk portion 220. Disk portion 220 prevents the blood from exiting hub 295.

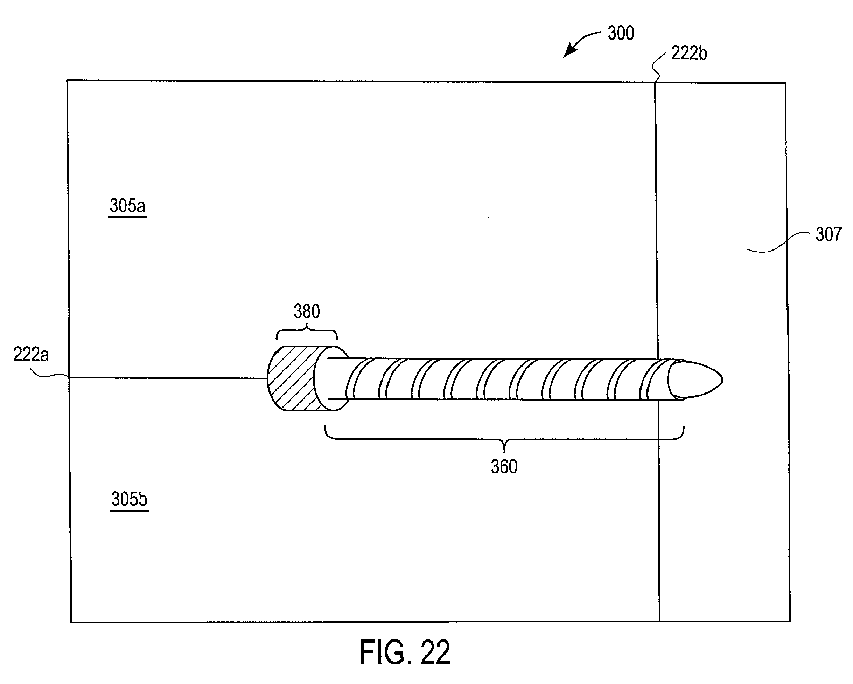

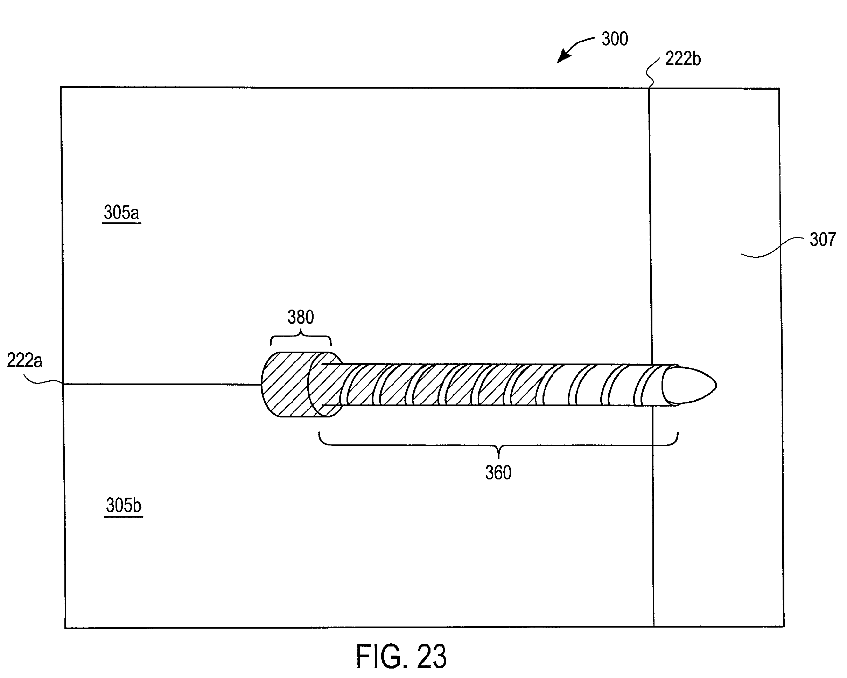

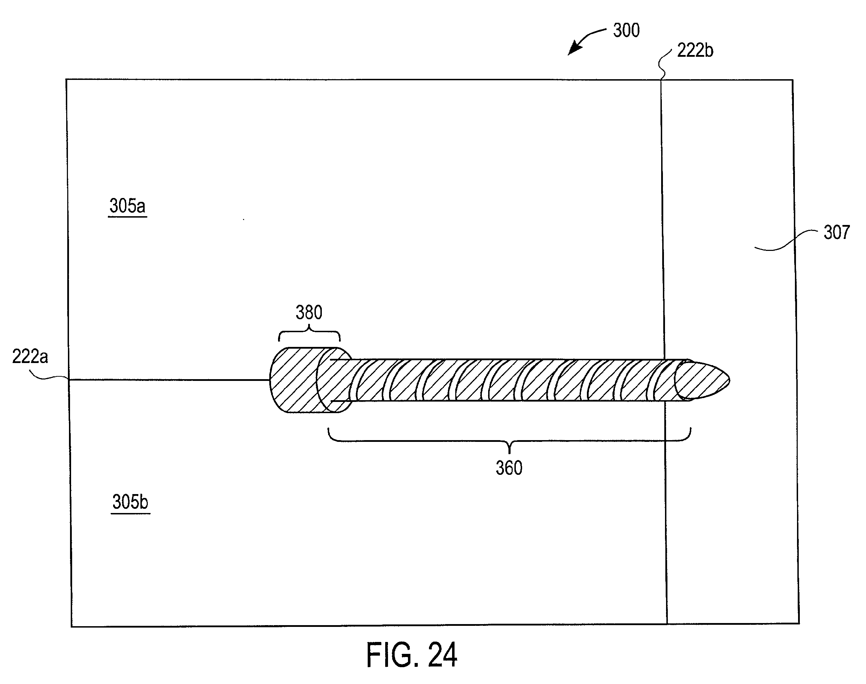

**Figure 22** illustrates mold 300 used to form a one-piece spiral needle in accordance with one embodiment of the invention. Mold 300 comprises first and second sections 305A and 305B and third section 307 that are mated together. The cavity formed by mold 300 comprises a proximal (or head) portion 380 and the distal portion 360. The distal portion 360 has spirals throughout the length of distal portion 360. In contrast, a proximal portion typically lacks spirals although it will be appreciated that it may have spirals in an alternate embodiment. It will be also be appreciated that the number of spirals depend upon the requirements of the health care worker. For example, the more spirals used, the more blood volume the needle can accommodate. This provides the healthcare worker with a better opportunity to observe flashback. The spirals are formed by having sections of mold 300 extend toward the center of the cavity used to form the one-piece needle. From the center of the cavity, the diameter of the recesses may range from 0.03 to 0.2 inches. When mold 300 is opened, the one-piece needle that is formed has spiral recesses where sections of mold 300 had previously been located. **Figure 22** further illustrates molten steel entering mold 300 at inlet 222a. Molten steel is introduced at inlet 222a of mold 300 at a pressure in the approximate range of 1 psi to 100 psi. Additionally, the molten steel is generally maintained at a temperature that ranges from 1600°F to 2200°F. It will be appreciated that other pressures and temperatures may be used depending upon the material selected. **Figure 23** illustrates that the steel has moved through head 380 in the general direction of the beveled distal tip of distal portion 360. **Figure 24** illustrates the molten steel having filled mold 300. It will be appreciated that the one-piece needle that is formed may have a tip that is solid or hollow bored. Additionally, the one-piece needle may have a groove portion at the top portion of the one-piece needle.

The mold opens at parting lines 222a and 222b. Once the mold is opened, the one-piece introducer needle is then removed from mold 300 using conventional techniques.

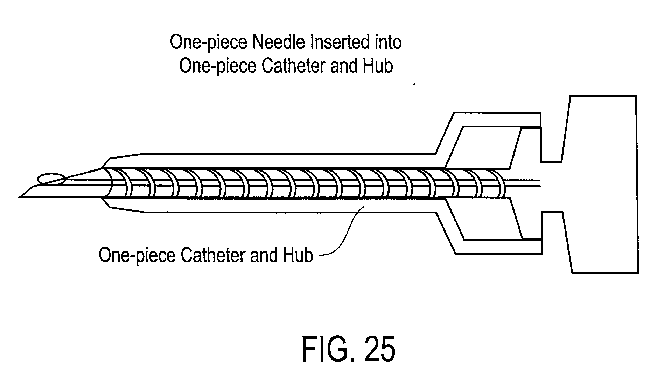

**Figure 25** illustrates the one-piece spiral needle inserted into a catheter and hub. The hub is located at 395. The diameter of the one-piece spiral needle and hub may range from 0.15 inches to 0.35 inches. This large range accommodates the design of a luer lock feature in the hub. The number of spirals that may be used for the lowest to the highest gauge may range from 5 to 15.

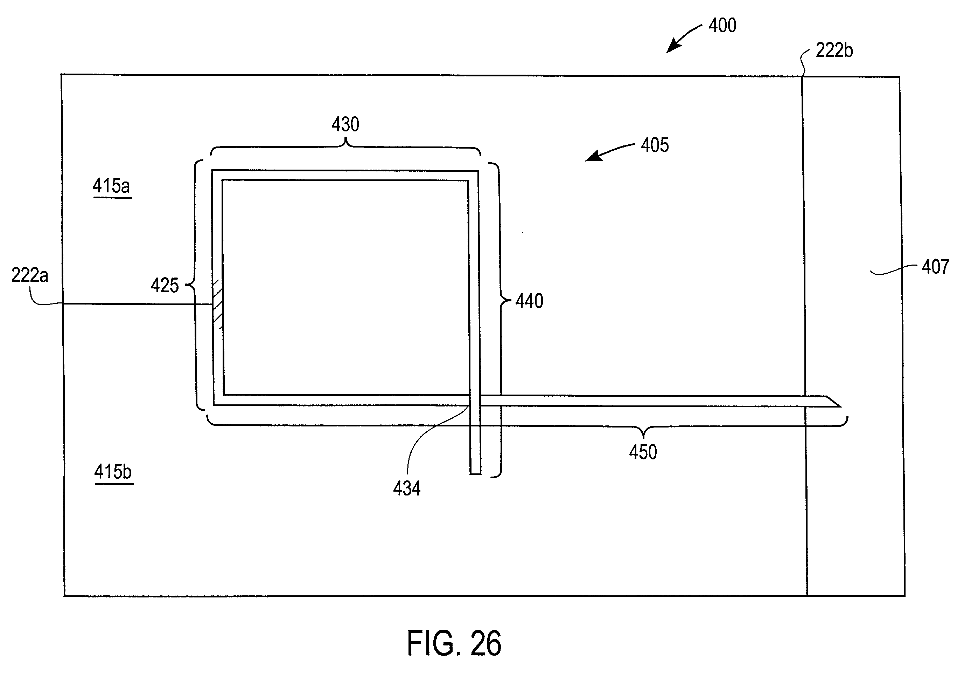

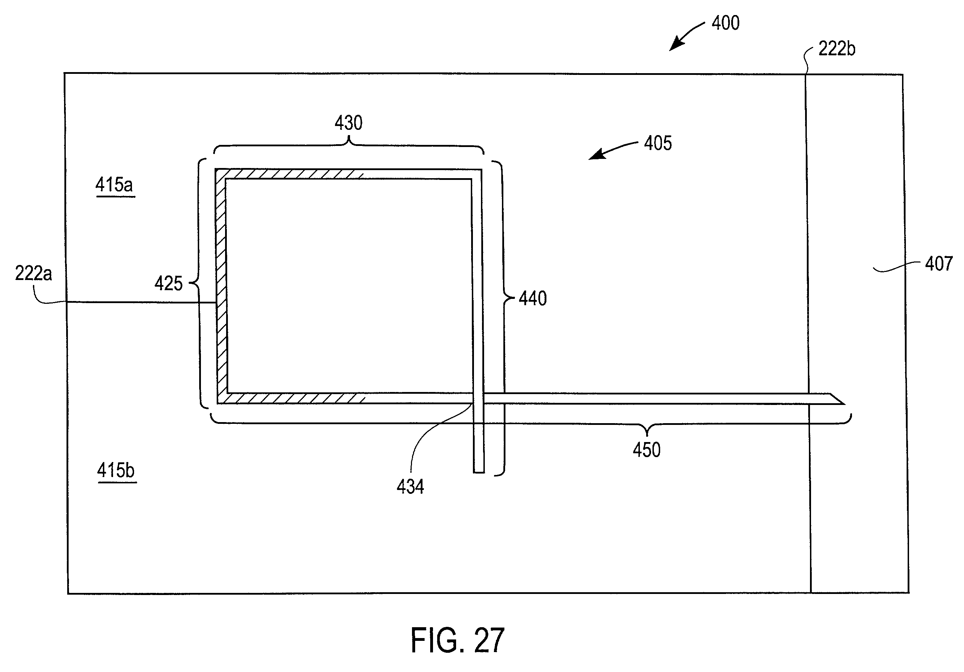

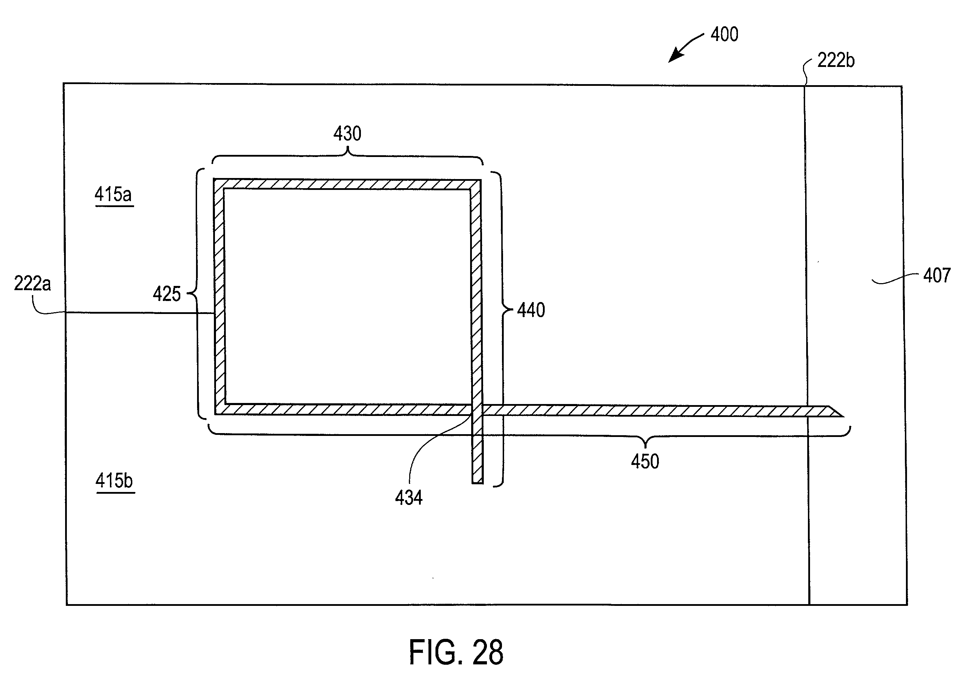

**Figures 26** through **29** illustrate one embodiment of the invention using injection molding to form a one-piece needle. **Figure 26** illustrates another mold used to form a one-piece needle in accordance with one embodiment of the invention. Mold 400 comprises first and second sections 415A and 415B and third section 407 that are mated together. The cavity formed by mold 400 comprises a substantially rectangular portion head (or handle) shaped head 425 and a distal portion 450 of the one-piece needle. It will be appreciated that molten steel enters at inlet 222a and travels in the direction of portions 430 and 450. At point 434, portion 440 does not communicate with portion 450. Instead, portion 440 of mold 400 overhangs portion 450. Therefore, the molten steel in portion 440 does not contact the molten steel in portion 450. **Figure 27** illustrates the molten steel using the same mold as **Figure 26** entering portion 440 and continuing in the direction of the distal portion of portion 450. **Figure 28** illustrates that the molten steel has filled the cavity of mold 400. It will be appreciated, however, that the distal portion of the one-piece needle may be made with the hollow needle tip or solid needle tip with a surface groove.

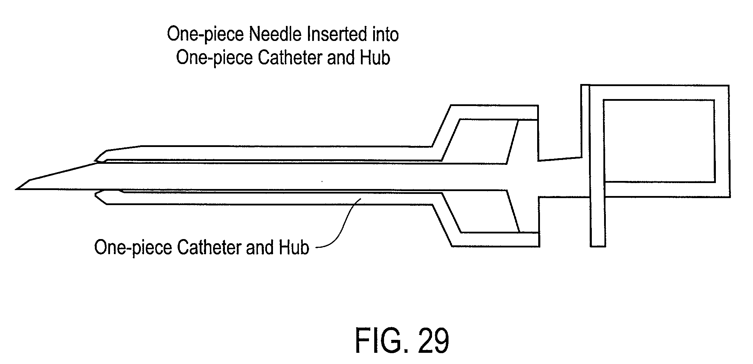

The mold opens at parting lines 222a and 222b. Once the mold is opened, the one-piece introducer needle is then removed from mold 400 using conventional techniques. **Figure 29** illustrates the one-piece needle formed from the process illustrated in **Figures 26-29** locked in place by using a catheter and hub that has a recessed region for receiving portion 440 of the one-piece needle.

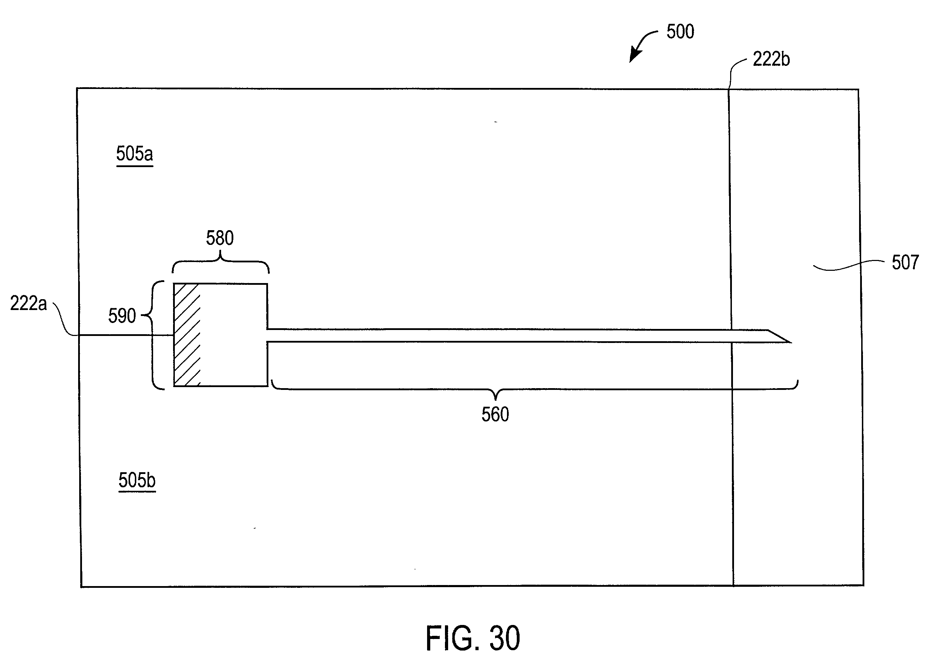

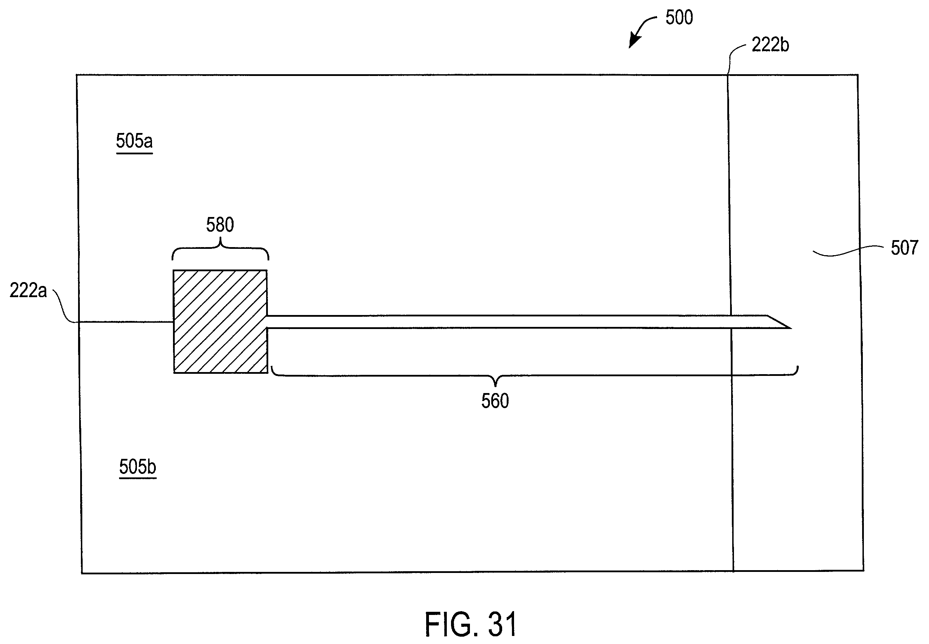

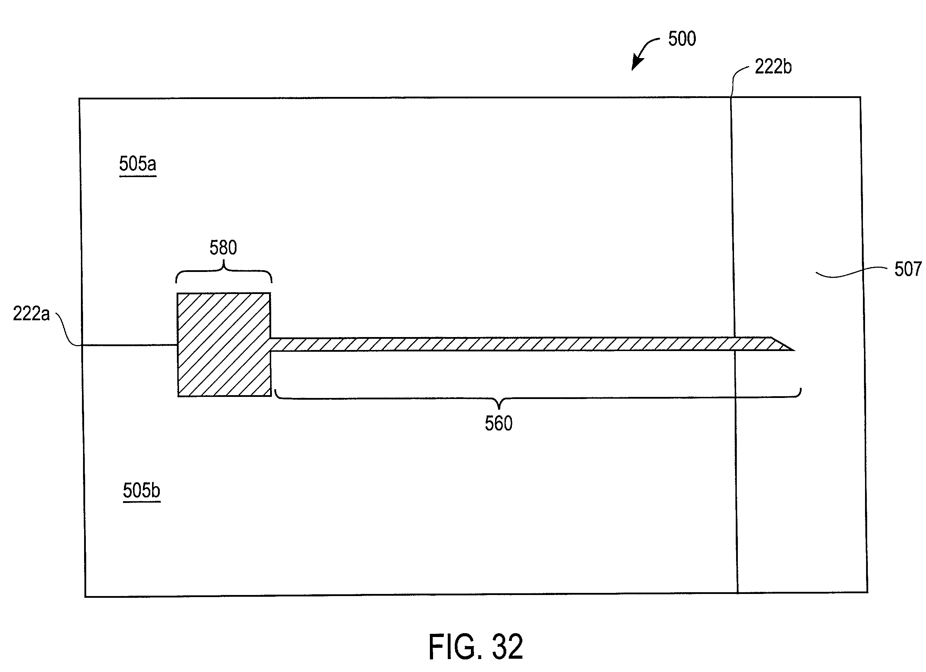

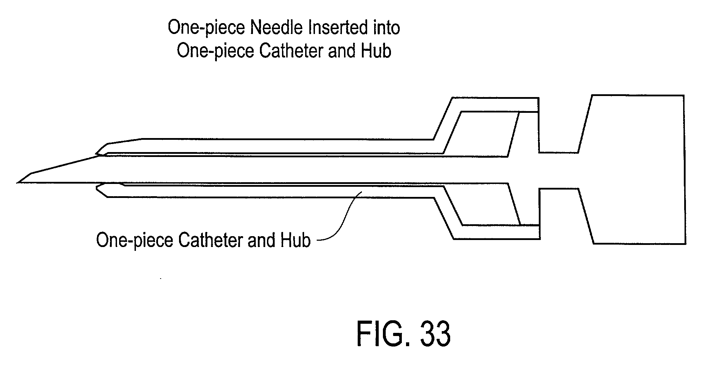

**Figures 30** through **33** illustrate mold 500 for forming a one-piece needle with a head portion in accordance with one embodiment of the invention. In **Figure 30,** mold 500 comprises first and second sections 505A and 505B and third section 507 mated together. The cavity formed by mold 500 comprises a substantially cylindrical or substantially square head at the proximal end formed by segments 580 and 590. Mold 500 has an inlet 222a that allows molten steel to enter mold 500. Section 580 may range in length from 0.5 inches to 2.0 inches. The length and diameter of needle portion 560 depends upon the gauge of the needle used as described above. **Figure 31** shows the molten steel has filled the head portion of the one-piece needle. **Figure 32** illustrates the same mold as **Figure 31** with the molten steel having filled the cavity of the one-piece needle. The mold opens at parting lines 222a and 222b. Once the mold is opened, the one-piece introducer needle is then removed from mold 500 using conventional techniques. In another embodiment, after the one-piece introducer needle has been formed, a metallic needle tip may be secured, attached, coupled, or fastened to the distal tip of the needle using conventional techniques. **Figure 33** illustrates the one-piece needle formed in **Figures 30-33** inserted into a hub and catheter.

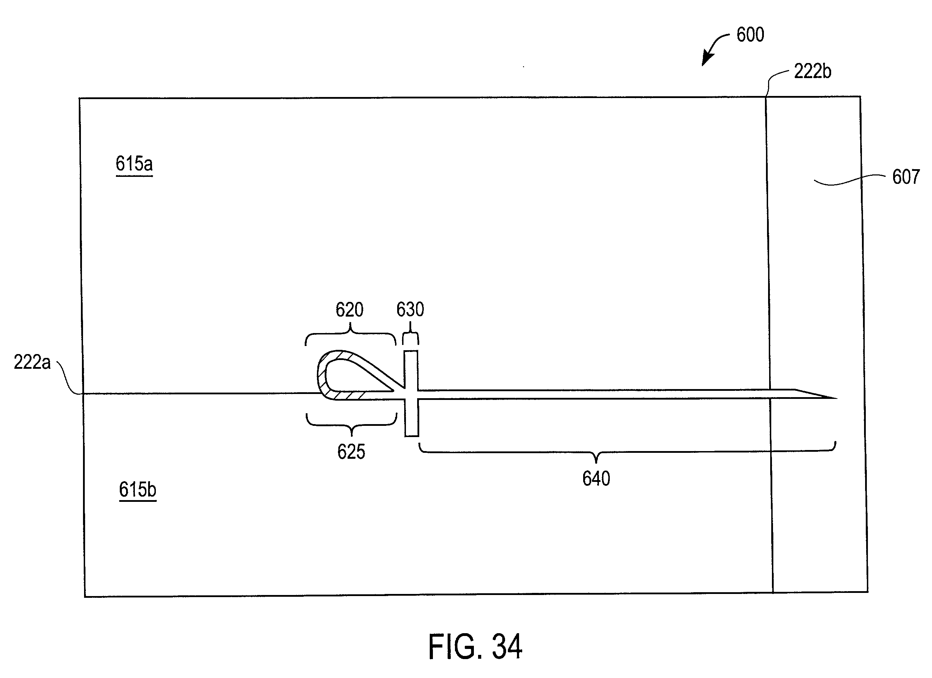

**Figure 34** illustrates mold 600 comprising first and second sections 615a and 615b and third section 607 that are mated together. The cavity formed by mold 600 comprises a head that has a straight portion 625 and an angled portion 620 that are joined together at disk portion 630. Needle portion 640 is the cavity used to form the needle. Mold 600 has an inlet 222a that allows molten steel to enter mold 600. The molten steel is introduced at inlet 222a of mold 600 at a pressure in the approximate range of 1 psi to 100 psi. Additionally, the molten steel is generally maintained at a temperature that ranges from 1600°F to 2200°F. It will be appreciated that other pressures and temperatures may be used depending upon the materials selected. The molten steel begins to move through straight portion 625 and angled portion 620. Straight portion 625 has a length that ranges between 0.5 to 2.0 inches. Angled portion 620 generally has a radius that ranges from approximately 0.4 to 1.0 inches.

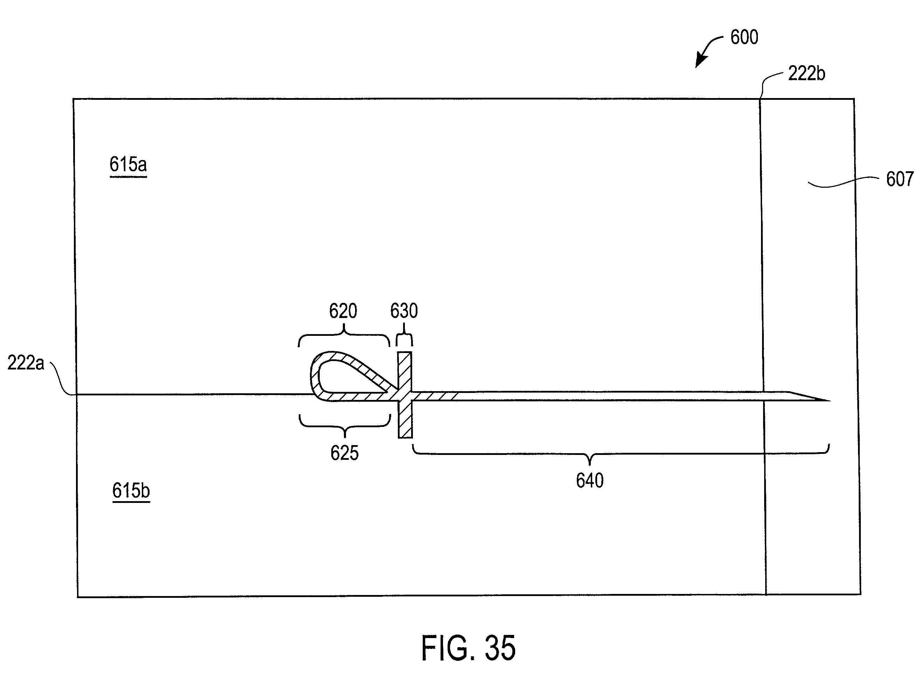

**Figure 35** illustrates mold 600 of **Figure 34** wherein the molten steel has advanced into needle portion 640.

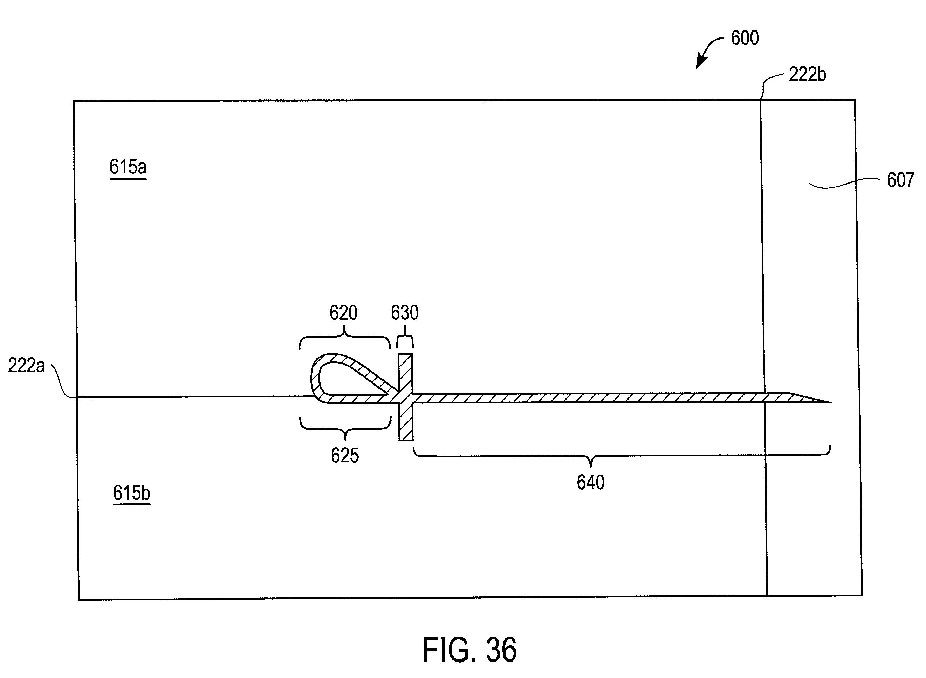

**Figure 36** illustrates mold 600 of **Figure 35** in which the mold is filled with the molten steel thereby forming a one-piece introducer needle. The mold opens at parting lines 222a and 222b. Once the mold is open, the one-piece introducer needle is then removed from mold 600 using conventional techniques. In another embodiment, after the one-piece introducer needle has been formed, a metallic needle tip may be secured, attached, coupled, or fastened to the distal tip of needle portion 640 using conventional techniques.

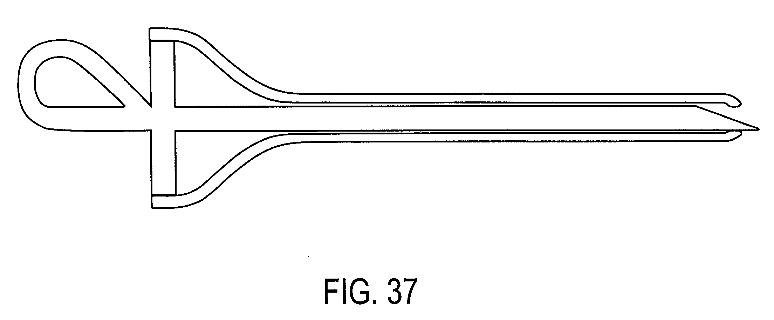

**Figure 37** illustrates the one-piece needle formed from the process shown in **Figure 34-36** inserted into a catheter and hub. It will be appreciated that the one-piece needle tip formed from this process may be either hollow or solid. Additionally, the one-piece needle may have a surface groove. The surface groove may have a variety of shapes as illustrated in **Figures 6-16, 22-25,** and **38-48.**

It will be appreciated that each mold described herein may be configured such that a surface groove is formed in the one-piece needle. **Figures 6** through **16, Figures 22** through **25,** and **Figures 38** through **48** illustrate some of the surface grooves formed from the molds described herein and the flow of blood through the surface grooves.

Given the variety of molds that may be used to form one-piece needles that may have surface grooves, **Figures 38** through **48** illustrate a variety of surface grooves in the one-piece needles formed by using techniques described herein. **Figure 38** illustrates a cross-section of a solid tip needle with a surface groove in accordance with an embodiment of the invention. Surface groove 60 is substantially circular in shape and is located in the wall of needle 40 near the outer diameter of needle 40. The surface groove has a depth that ranges from 0.001 inches to 0.05 inches depending upon the needle gauge. It will be appreciated that surface groove 60 generally extends the length of needle 40 or any portion thereof.

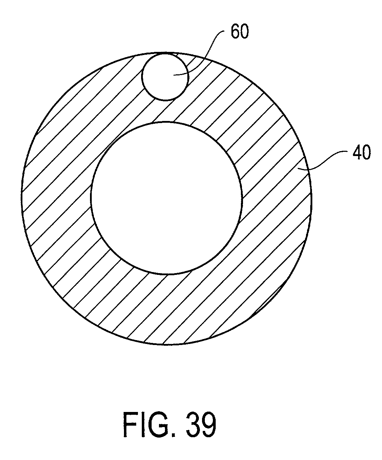

**Figure 39** illustrates a cross-section of a hollow bored tip needle with a surface groove in accordance with an embodiment of the invention. Surface groove 60 is substantially circular in shape and is located in the wall of needle 40 near the outer diameter of needle 40. The diameter of the surface groove ranges from 0.001 inches to 0.05 inches depending upon the needle gauge. It will be appreciated that surface groove 60 generally extends the length of needle 40 or any portion thereof.

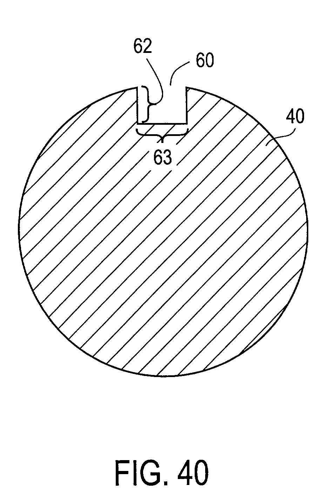

In yet another embodiment of the invention, **Figure 40** illustrates a cross-section of a solid tip needle with a substantially rectangular portion shaped surface groove with one side that is open. Surface groove 62 ranges from 0.001 inches to 0.008 inches and the width 63 ranges from 0.001 inches to 0.05 inches both depending upon the needle gauge. Surface groove 60 allows blood to enter at the distal end of surface groove 60.

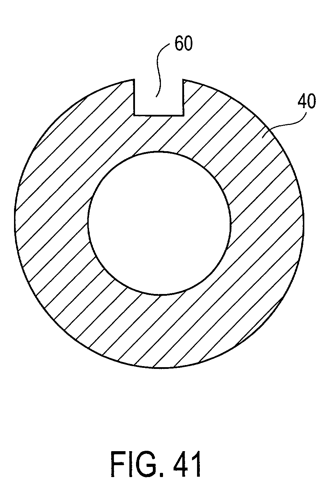

In another embodiment of the invention, **Figure 41** illustrates a cross-section of a hollow bored tip needle with a substantially rectangular portion shaped surface groove with one side that is open. The dimensions of the surface groove include a length that ranges from 0.001 inches to 0.05 inches, a height that ranges from 0.001 inches to 0.05 inches, and a width that ranges from 0.001 inches to 0.05 inches all depending upon the needle gauge. Surface groove 60 allows blood to enter at the distal end of surface groove 60 or through the bore of needle 40 and then into surface groove 60.

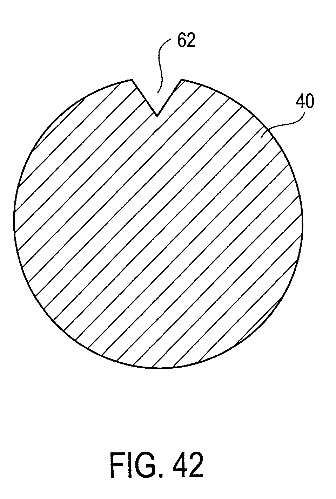

**Figure 42** illustrates a cross-section of a solid tip needle with a surface groove having a substantially v-shape in accordance with an embodiment of the invention. Each side that makes up the v-shape ranges from 0.001 inches to 0.05 inches depending upon the needle gauge. With this shape of a surface groove, blood flow will have an increased rate of speed at the outer ends of the v-shaped surface groove and a slower rate of movement at central end of v-shaped surface groove.

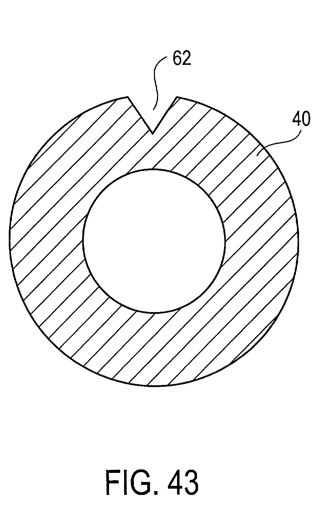

**Figure 43** illustrates a cross-section of a hollow bored tip needle with a surface groove having a substantially v-shape in accordance with an embodiment of the invention. The dimensions of each side of the v-shape are the same as in **Figure 38.** Blood will generally flow through the bore and into surface groove 62.

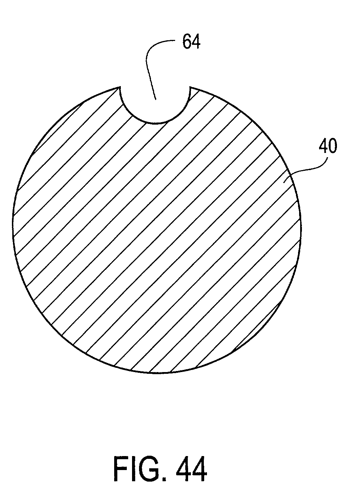

**Figure 44** illustrates a cross section of a solid tip needle with one surface groove 64 having a substantially half-circle shape in accordance with an embodiment of the invention. The surface groove has the depth approximately in the range of 0.001 inches to 0.05 inches depending upon the needle gauge. Surface groove 64 is formed at the outer surface of needle 40. Surface groove 64 may extend the length of needle 40 or any portion thereof. Because needle 40 is solid, blood flows around the side of needle 40 into and through surface groove 64.

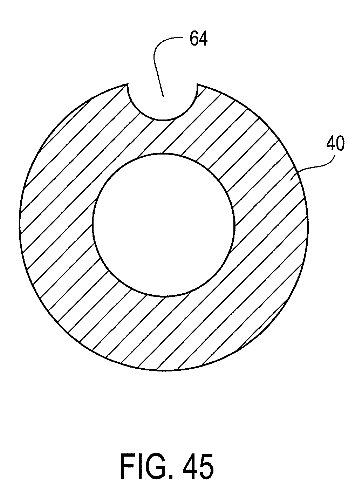

**Figure 45** illustrates a cross-section of a hollow bored tip needle 40 having a substantially half circle shape surface groove 64 in accordance with an embodiment of the invention. The depth of the groove ranges from 0.001 inches to 0.05 inches depending upon the needle gauge. Since needle 40 has a hollow bore, blood may flow into the bore and then into surface groove 64.

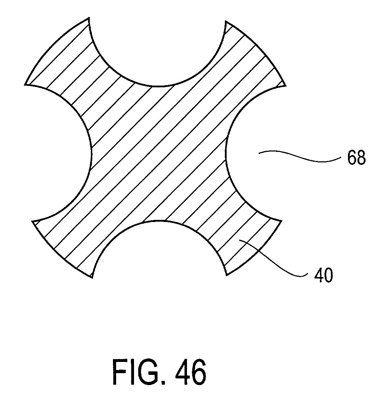

In yet another embodiment of the invention, **Figure 46** illustrates a plurality of substantially half-circle surface grooves 68 in solid tip needle 40. The depth of each substantially half circle ranges from 0.001 inches to 0.05 inches depending upon the needle gauge. Blood or other bodily fluids flow through surface groove 68.

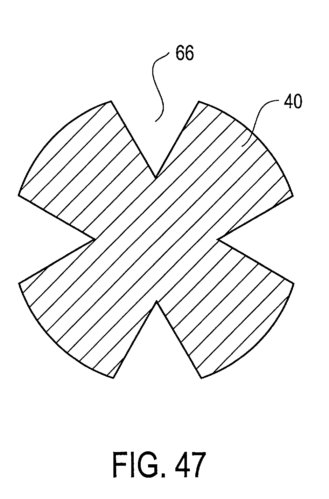

**Figure 47** illustrates a plurality of substantially v-shaped surface grooves 66 in accordance with an embodiment of the invention. Each surface groove may extend the entire length of needle 40 or any portion thereof. As noted previously, the flow of blood travels at an increased rate at the outer ends of the v-shape and flows more slowly at the central portion of the v-shape surface groove. Each side of the v-shape surface groove ranges from 0.001 inches to 0.05 inches depending upon the needle gauge.

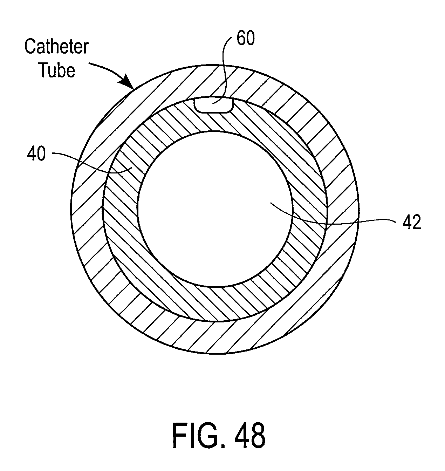

In yet another embodiment of the invention, **Figure 48** illustrates a cross-sectional view of needle 40 coaxially nestled into blunting member 42 in accordance with one embodiment of the invention. Blunting member 42 is made of substantially rigid material such as plastic or metal. Needle 40 has a surface groove 60 that is substantially rectangular in shape. One side of the substantially rectangular shape ranges from 0.001 inches to 0.05 inches and the other side ranges from 0.001 inches to 0.005 inches both depending upon the needle gauge. It will be appreciated that other shapes for a surface groove may be used such as a v-shape, a half circle shape, and any other suitable shape. Surface groove 60 allows blood to enter at the distal end of surface groove 60 or through the bore of needle 40 and then into surface groove 60.

In the preceding detailed description, the invention is described with reference to specific embodiments thereof. It will, however, be evident that various modifications and changes may be made thereto without departing from the broader spirit and scope of the invention as set forth in the claims. The specification and drawings are, accordingly, to be regarded in an illustrative rather than a restrictive sense.

## Claims

1. A method of forming a one-piece needle having a head and a needle, comprising:
feeding a molten steel into a cavity of a mold, wherein the first portion of the cavity is a head and the second portion of the cavity is a needle and a surface groove.

2. The method of claim 1, further comprising:
forming a one-piece needle having a surface groove.

3. The method of claim I or claim 2, further comprising ejecting the one-piece needle.

4. The method of any one of claims 1 to 3, wherein the steel is introduced into a mold at a temperature approximately in the range of 1600°F to 2200°F.

5. The method of claim 4, wherein the steel is introduced at a pressure approximately in the range of 1 psi to 100 psi.

6. The method of any one of claims 1 to 5, wherein the head of the cavity is tapered at the distal end of the head.

7. The method of any one of claims 1 to 6, wherein a tip of the needle is hollow or solid.

8. The method of any one of claims 1 to 7, wherein a tip of the needle is sharp.

9. The method of any one of claims 1 to 8, wherein the surface groove has a cross-section shape which is a substantially v-shape, substantially rectangular shape, substantially at least half of a circle shape, or a portion which is substantially curved.

10. The method of any one of claims 1 to 9, wherein the head has a substantially square shape, a substantially rectangular shape or a substantially cylindrical shape or is substantially at least half of a circle in shape.
